# EUROPEAN PATENT SPECIFICATION

(11) **EP 3 903 858 B1**
(45) Date of publication and mention of the grant of the patent: **27.11.2024**
(21) Application number: 19903005.7
(22) Date of filing: 27.12.2019
(51) Int. Cl.: A61M 5/30, A61M 5/303, A61M 5/315, A61M 5/20, A61M 15/08

(54) **NEEDLELESS INJECTOR**
NADELLOSER INJEKTOR
INJECTEUR SANS AIGUILLE

(30) Priority: 27.12.2018 JP 2018245622; 25.01.2019 JP 2019011478
(43) Date of publication of application: 03.11.2021
(73) Proprietor: Daicel Corporation, Osaka 530-0011 (JP)
(72) Inventor: SUZUKI, Takamasa, Tokyo 108-8230 (JP); HASEGAWA, Takashi, Tokyo 108-8230 (JP); ATOBE, Shingo, Tokyo 108-8230 (JP); YAMAMOTO, Yuzo, Tokyo 108-8230 (JP); IGA, Hiromitsu, Tokyo 108-8230 (JP)
(74) Representative: Grünecker Patent- und Rechtsanwälte PartG mbB
(86) International application number: PCT/JP2019/051556
(87) International publication number: WO 2020/138476

(56) References cited:
- GB-A- 2 187 962
- JP-A- 2009 056 339
- JP-A- H10 512 165
- JP-B2- 6 215 582

## Description

### Technical Field

The present disclosure relates to a needleless injector that ejects an intended injection substance to a target region without using an injection needle.

### Background Art

JP 6 215 582 B2 shows a needleless injector having the features according to the preamble of claim 1.

An example of a device that ejects a liquid chemical to a target region such as an organism includes a needleless injector using no injection needle which has been attracting attention in terms of usability and sanitation, and thus has been actively developed recently. In general, there has been implemented a needleless injector having a configuration in which a liquid chemical pressurized by a driving source such as compressed gas and a spring is ejected to a target region and the liquid chemical is administered to an inside of the target region through use of the kinetic energy of the liquid chemical.

The needleless injector adopts a configuration in which the intended injection substance is pressurized and imparted with ejection energy to be administered into the target region. Thus, when the intended injection substance is ejected, an ejection port of the needleless injector is appropriately arranged with respect to the target region, and is preferably placed in a state of being in contact with the surface of the target region. For example, the needleless injector disclosed in Patent Document 1 has a holder that is disposed on a distal end side of the injector main body and holds a nozzle defining a flow path, in which the intended injection substance flows, and the ejection port. The distal end surface of the nozzle and the distal end surface of the holder may be flush, or one of the distal end surfaces may be more on the distal end side than the other. The needleless injector disclosed in Patent Document 2 is configured with an ejection unit, defining a flow path on the distal end side, loaded in a housing. The needleless injector thus configured has the distal end surface of the ejection unit, in which the ejection port is formed, arranged slightly more on the distal end side than the distal end surface of the housing.

### Citation List

### Patent Document

Patent Document 1: JP 2012-161431 A
Patent Document 2: JP 2013-146452 A

### Summary of Invention

### Technical Problem

The needleless injector ejects the intended injection substance, imparted with the ejection energy, to the target region through the ejection port, whereby the intended injection substance can be administered. Thus, a contact state between the ejection port and the target region while the injection is being performed largely affects the administration performance of the needleless injector. In view of this, the configuration of the distal end side of the needleless injector on which the ejection port is formed can be regarded as an important configuration affecting the administration performance of the needleless injector. The needleless injector according to the related art has a configuration in which an enclosure, such as the holder or the housing, accommodates a member such as the ejection unit and the nozzle that is connected to the ejection port and defines the flow path in which the intended injection substance imparted with the ejection energy flows, with only the distal end surface of the member in which the ejection port is formed exposed.

The related art of such a mode featuring the enclosure accommodating the member involves a risk of compromising the operability of the needleless injector because the enclosure is likely to interfere with the target region. As described above, to improve the administration performance of the needleless injector, the distal end surface of the member in which the ejection port is formed and the surface of the target region are required to be favorably in contact with each other. Unfortunately, when the target region is likely to be deformed by external pressing force as in the case of an organism or the like, the enclosure described above is likely to interfere with the target region. To favorably administer the intended injection substance into the target region, the needleless injector needs to be appropriately fixed to the target region despite the impact, vibrations, or the like produced by the ejection. The fixing requires pressing force that is likely to result in the above described interference between the enclosure and the target region.

In view of the above, an object of the present application is to provide a technique with which a favorable contact state between the ejection port and the target region is achieved and high operability of the needleless injector may be maintained.

### Solution to Problem

To solve the problem described above, a needleless injector according to claim 1 is provided. Preferred embodiments of the invention are defined in the dependent claims.

### Advantageous Effects of Invention

A suitable contact state between an ejection port and a target region can be achieved, and high operability of the needleless injector can be maintained.

### Brief Description of Drawings

FIG. 1 is a diagram illustrating a schematic configuration of a needleless injector.
FIG. 2 is a first cross-sectional view of a needleless injector.
FIG. 3 is a second cross-sectional view of the needleless injector.
FIG. 4 is a diagram illustrating a configuration of a housing of the needleless injector.
FIG. 5 is a diagram illustrating a schematic configuration of an injector assembly incorporated in the needleless injector.
FIG. 6 is a diagram illustrating a schematic configuration of an actuator incorporated in the needleless injector.
FIG. 7 is a diagram illustrating a schematic configuration of a piston incorporated in the needleless injector.
FIG. 8 is a diagram illustrating a schematic configuration of an attachment incorporated in the needleless injector.
FIG. 9 is a diagram illustrating a schematic configuration of a plunger rod and plunger incorporated in the needleless injector.
FIG. 10 is a diagram illustrating a schematic configuration of a container incorporated in the needleless injector.
FIG. 11 is an enlarged view of a distal end side of the container illustrated in FIG. 10.

### Description of Embodiments

With reference to the drawings, a needleless injector 1 according to an embodiment of the present disclosure (herein, simply referred to as "injector") is described below. The injector 1 is a needleless injector that implements injection by ejecting an ejection solution, which corresponds to an intended injection substance in the present application, to a target region through use of a combustion energy of an explosive, that is, a device that injects the ejection solution to the target region without using an injection needle.

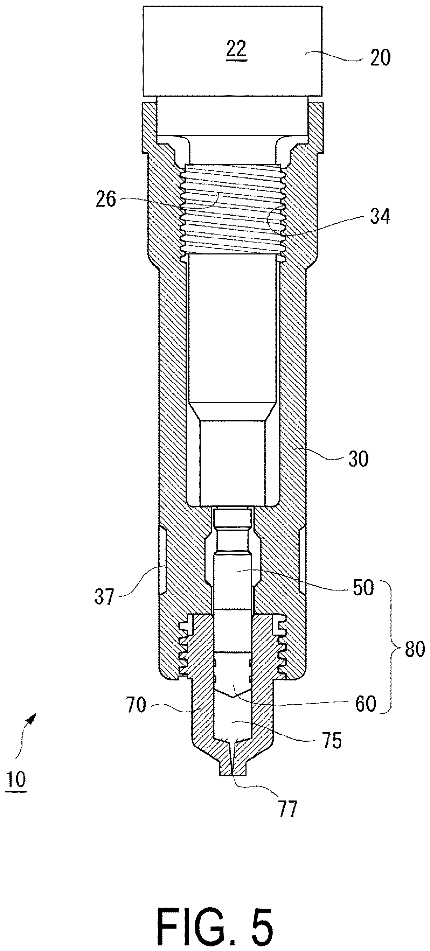

Note that each of the configurations, combinations thereof, and the like in each embodiment are an example, and various additions, omissions, substitutions, and other changes may be made as appropriate without departing from the spirit of the present disclosure. The present disclosure is not limited by the embodiments and is limited only by the claims. Note that, in the present embodiment, as terms indicating a relative positional relationship in a longitudinal direction of the injector 1, "distal end side" and "base end side" are used. The "distal end side" indicates a side closer to the distal end of the injector 1 described later, that is, a position closer to an ejection port 77, and the "base end side" indicates a side in an opposite direction to the "distal end side" in a longitudinal direction of the injector 1, that is, a direction to an igniter 22 side of an injector assembly 10 (see FIG. 5 described later).

### Configuration of Injector 1

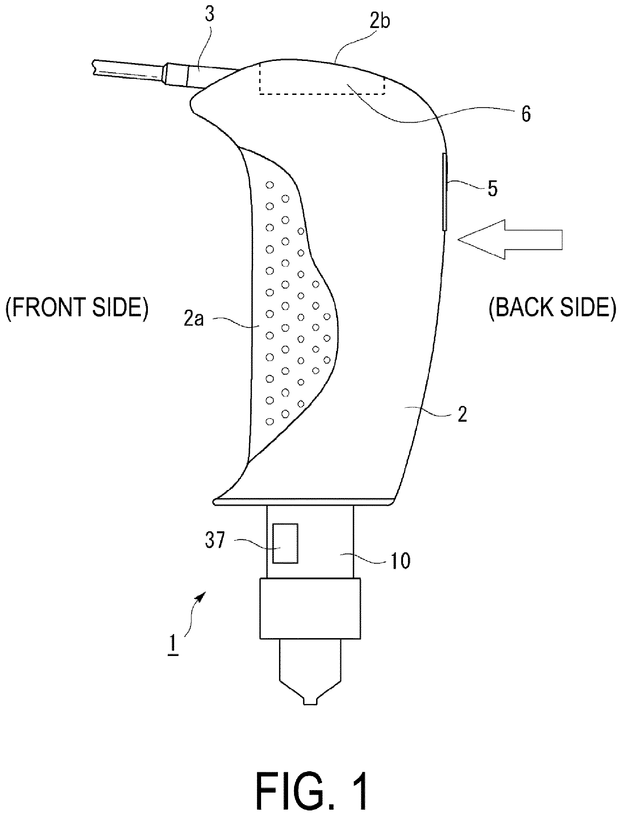

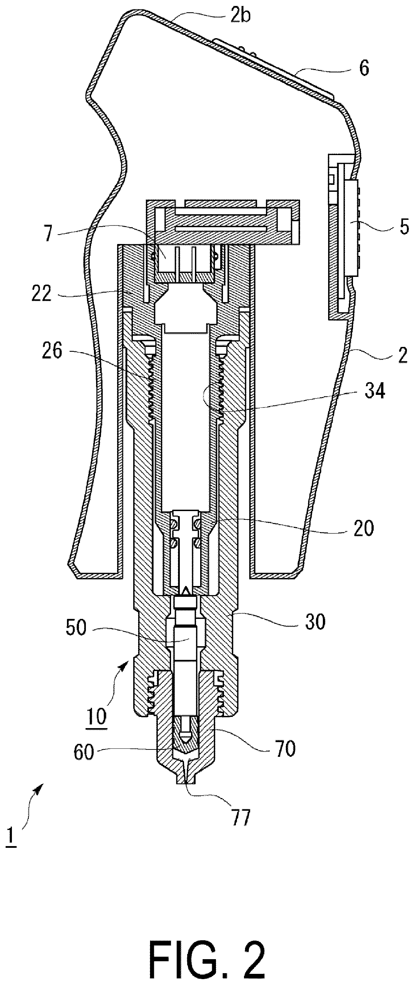

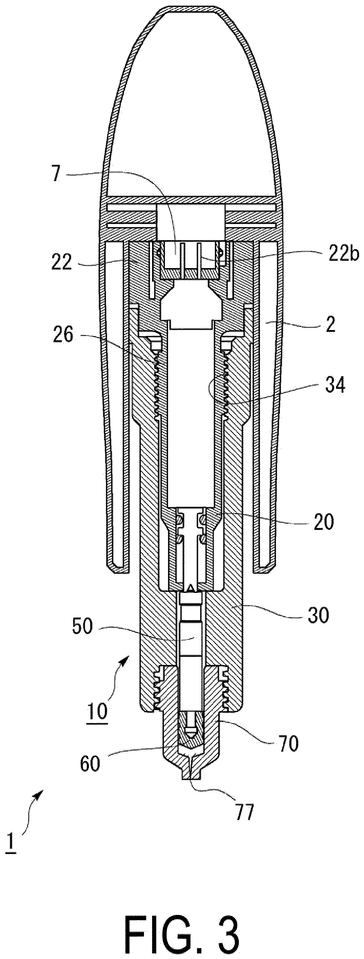

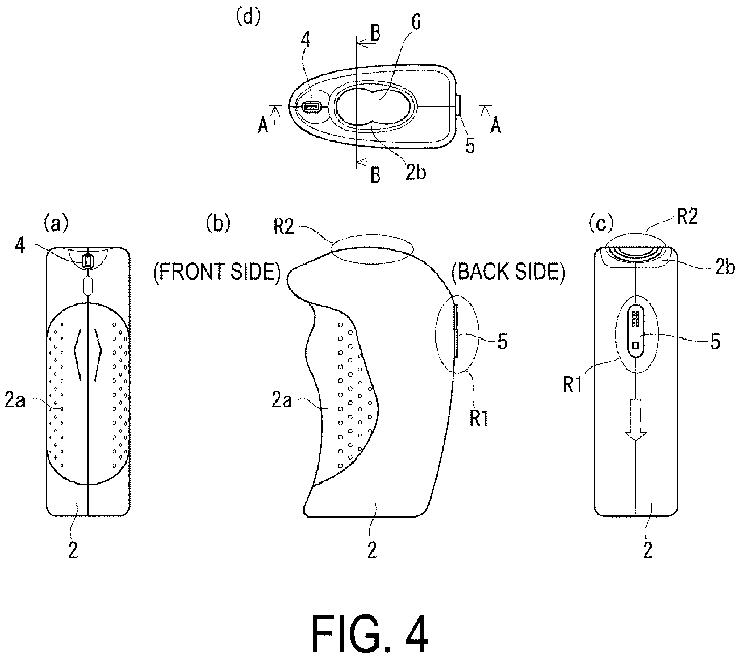

Here, FIG. 1 is a diagram schematically illustrating the appearance of the injector 1. FIG. 2 is a first cross-sectional view of the injector 1, which is an AA cross section in FIG. 4, described below. FIG. 3 is a second cross-sectional view of the injector 1, a BB cross section in FIG. 4 described below. The BB cross section is orthogonal to the AA cross section. Note that FIG. 4 is a diagram illustrating a configuration of a housing 2 that is a part of the injector 1. Here, the injector 1 is formed with the injector assembly 10 attached to the housing 2 (injector enclosure). A power cable 3 for supplying drive current to the igniter 22 in the injector assembly 10 is connected to the housing 2.

Note that, in the following description in the present application, the ejection solution ejected to the target region by the injector 1 is formed of a liquid medium including a predetermined substance, which exerts an effect or a function expected in the target region. In the ejection solution, the predetermined substance may be in a state of being dissolved in liquid being a medium, or may be in a state of being simply mixed, rather than being dissolved.

For example, examples of the predetermined substance included in the ejection solution include an organism-derived substance and a substance with a desired bioactivity, which can be ejected to the target region being an organism. For example, examples of the organism-derived substance include DNA, RNA, a nucleic acid, an antibody, and a cell. Examples of the substance with a bioactivity include various substances exerting pharmacological or therapeutic effects, which are exemplified by medicines composed of low molecular compounds, proteins, peptides, or the like, a vaccine, an inorganic substance such as metal particles for thermotherapy or radiotherapy, and a carrying body functioning as a carrier. Further, the liquid as the medium of the ejection solution is only required to be a substance suitable for administering the predetermined substance as exemplified above to the target region, and may be aqueous or oleaginous, which is not limited. Further, viscosity of the liquid as the medium is not particularly limited as long as the predetermined substance can be ejected by the injector 1.

In the injector 1, the injector assembly 10 is freely attachable to and detachable from the housing 2. An accommodating space 75 (see FIG. 5) formed between a container 70 and a plunger 80 in the injector assembly 10 is filled with ejection solution during a preparation stage before the operation of the injector 1. The injector assembly 10 is a unit that is replaced each time the ejection solution is ejected. The injector assembly 10 will be described in detail below.

On the other hand, the housing 2 has a grip portion 2a formed to be gripped by a user of the injector 1 in use, and is provided with a plurality of switches for operating the injector 1 to eject the ejection solution. Note that the injector 1 is configured to be capable of being held and operated by one hand of the user. In this context, the housing 2 will be described with reference to FIG. 4. In FIG. 4, (a) illustrates the outer appearance of the housing 2 as viewed from the front side, (b) illustrates the outer appearance of the housing 2 as viewed from one side, (c) illustrates the outer appearance of the housing 2 as viewed from the back side, and (d) illustrates the outer appearance of the housing 2 as viewed from the upper side. Here, "front side" indicates a portion positioned on the distal side of the user holding the housing 2, which is the left side in FIG. 4(b), and "back side" indicates a portion positioned on the proximal side of the user holding the housing 2, which is the right side in FIG. 4(b). The "front side" and "back side" as illustrated in FIG. 4(b) respectively match the "front side" and "back side" illustrated in FIG. 1. Thus, when the user holds the housing 2 with one hand, fingertips rest on the front side of the housing 2 which is the distal side, and the wrist is in the vicinity of the back side of the housing 2 which is the proximal side. The "upper side" is a portion of the injector 1 on the base end side.

Considering such a way of holding by the user, the grip portion 2a is provided at a front side portion of the housing 2 such that the user can easily rest user's fingertips thereon. The grip portion 2a is provided with a plurality of dimples, making the user's fingertips even easier to be rested thereon. Furthermore, the grip portion 2a has gentle recesses and protrusions on the front side of its outer shell (see (b) in FIG. 4) such that the user's forefinger and middle finger can be easily rested thereon, for the sake of more stable holding of the housing 2 by the user.

Further, the housing 2 is provided with a first switch 5 and a second switch 6 that are two operating switches for operating the injector 1. The first switch 5 and the second switch 6 are connected to an unillustrated control unit, formed by a microcomputer. The control unit controls the supply of ignition current to the igniter 22 based on a signal transmitted from each switch, thereby controlling an operation of the injector 1. The first switch 5 is a sliding switch provided in a first region R1 in a side surface on the back side of the housing 2, the sliding direction of which being an upward and downward direction of the housing 2 (direction between the distal end and the base end, as indicated by a white arrow in FIG. 4(c)). The first region R1 is a region covered by the palm (a portion around the base of the thumb) of the user who attempts to hold the housing 2 with one hand. Thus, when the user holds the housing 2, the palm of the hand comes into contact with the first switch 5 while covering the first switch 5 from the above. The contact direction of the palm, that is, a direction in which the palm approaches the first switch 5 (a direction indicated by a white arrow in FIG. 1) is a direction different from the sliding direction (a direction toward the distal end of the injector 1) that is an operation direction of the sliding first switch. When the user performs a sliding operation on the first switch 5, user's palm is partially separated from the first region R1 temporarily with some of the fingers (the forefinger or the middle finger for example) resting on the housing 2 while enabling a remaining finger (the thumb for example) access the first switch 5, and the sliding operation can be performed on the first switch 5 using the remaining finger.

The first switch 5 is constantly biased in the upward direction. When the user continuously slides the first switch 5 downward (toward the distal end side) for a predetermined period of time against the biasing force, the control unit detects the sliding state of the first switch 5 and can place the injector 1 in a standby state. The standby state is a state in which preparation for the injector 1 to eject the ejection solution has been completed. When a user makes an additional operation (an operation on the second switch 6 described later) in this state, the ejection is implemented. The first switch 5 configured as described above is an operation switch for placing the injector 1 in the standby state. The contact direction of the palm of the user to hold the housing 2 is different from the operation direction of the first switch 5. Thus, the first switch 5 can be prevented from receiving an unintentional sliding operation by the user who holds the housing 2.

The second switch 6 is a pressing switch provided in a second region R2 including an inclined surface 2b on the upper side of the housing 2. The user can press the second switch 6 in a direction toward the inner side of the housing 2. The second region R2 is a region that is exposed without being covered by the palm of the user who holds the housing 2. The second switch 6 has an upper side surface inclined toward the side of the user, together with the inclined surface 2b in the second region R2. Alternatively, the upper side surface of the housing 2 may form the inclined surface without the upper side surface of the second switch 6 inclining. When at least the upper side surface of the second switch 6 thus forms the inclined surface, the user who holds the housing 2 with one hand can more easily visually recognize the upper side surface, which may be the pressing portion of the second switch 6, and can perform the pressing operation on the second switch 6 in a natural holding state. The upper side surface of the second switch 6 is thus more easily recognizable by the user and the second switch 6 is thus able to be pressed in the natural holding state, which enables the user to stably operate the injector 1 while clearly recognizing the presence of the second switch such that erroneous operation can be prevented, and thus leads to more reproducible administration.

As described above, the control unit supplies an ignition current to the igniter 22 in response to the pressing operation on the second switch 6 while the injector 1 is in the standby state as a result of the operation on the first switch 5 described above. As a result, the injector 1 ejects the ejection solution. In view of this, the second switch 6 may be regarded as the operation switch of the injector 1 for determining the ejection of the ejection solution. Thus, the configuration in which the upper side surface of the second switch 6 is formed by an inclined surface to be more visible to the user as described above may be regarded as an extremely effective configuration in terms of stable operation of the injector 1.

A connector 4 to which the power cable 3 is connected is provided on the front side of the inclined surface 2b on the upper side surface of the housing 2. In the present embodiment, the connector 4 is a USB connector, and the power cable 3 is freely attachable to and detachable the housing 2. Alternatively, the power cable 3 may be a cable that is not detachable from the housing 2. In the present embodiment, the power for actuating the igniter 22 is supplied from the outside through the power cable 3. Alternatively, a battery for supplying such power may be provided inside the housing 2. In this case, the housing 2 can be repeatedly used while replacing the injector assembly 10, until the battery runs out. When the battery runs out, the battery may be replaced.

A schematic configuration of the injector assembly 10 is illustrated in FIG. 5. The injector assembly 10 is attached to the housing 2 to form the injector 1, as illustrated in FIGS. 2 and 3. Specifically, the injector assembly 10 is an assembly including an actuator 20, an attachment 30, the container 70, and the plunger 80. How the injector assembly 10 is assembled will be described below.

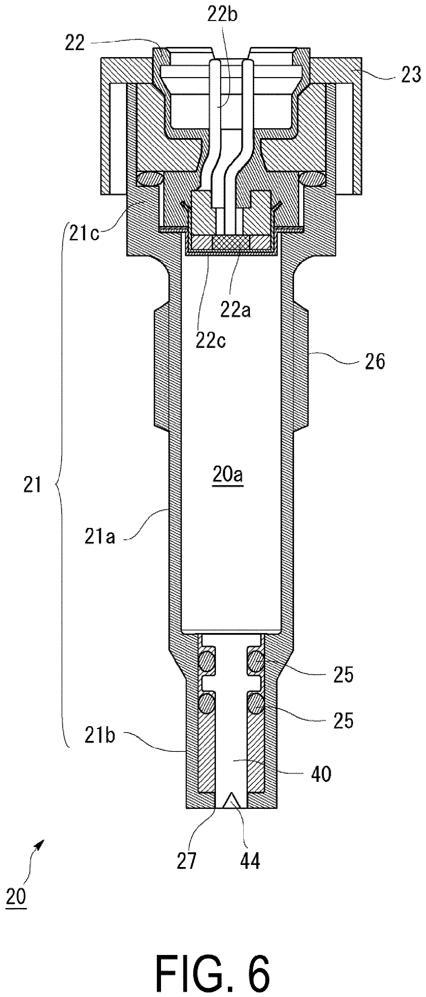

First of all, the actuator 20 will be described with reference to FIG. 6. The actuator 20 has a body 21 formed in a tubular shape. The body 21 includes a center portion 21a in the center thereof, a distal end portion 21b on the distal end side thereof, and a base end portion 21c on the base end side thereof. The distal end portion 21b, the center portion 21a, and the base end portion 21c of the body 21 have their internal spaces in communication with each other. The distal end portion 21b has an opening 27 on the distal end side. The igniter 22, which is an electric igniter that generates energy for ejection through combustion of an ignition charge 22a, is attached to the base end portion 21c of the body 21 via a cap 23. The igniter 22 has an ignition pin 22b to which ignition current is supplied from the outside. The ignition pin 22b is coupled to a socket 7 on the side of the housing 2 in a state in which the injector assembly 10 is attached to the housing 2. The attachment state of the igniter 22 to the body 21 is determined such that a combustion product generated by the operation of the igniter 22 is discharged toward the center portion 21a of the body 21. Specifically, the igniter 22 is attached to the base end portion 21c of the body 21 to have a discharge surface 22c, from which the combustion product is discharged, directed toward the center portion 21a.

Herein, a combustion energy used in the igniter 22 for the ignition charge is an energy for the injector 1 to eject the ejection solution to the target region. Note that, examples of the ignition charge include an explosive containing zirconium and potassium perchlorate (ZPP), an explosive containing titanium hydride and potassium perchlorate (THPP), an explosive containing titanium and potassium perchlorate (TiPP), an explosive containing aluminum and potassium perchlorate (APP), an explosive containing aluminum and bismuth oxide (ABO), an explosive containing aluminum and molybdenum oxide (AMO), an explosive containing aluminum and copper oxide (ACO), an explosive containing aluminum and iron oxide (AFO), or an explosive composed of a combination of a plurality of these explosives. These explosives exhibit characteristics that, although the explosives generate high-temperature and high-pressure plasma during combustion immediately after ignition, when the combustion product condenses at room temperature, the explosives do not contain gaseous components and hence the pressure generated decreases abruptly. An explosive other than these may be used as the ignition charge as long as appropriate ejection of the ejection solution can be performed.

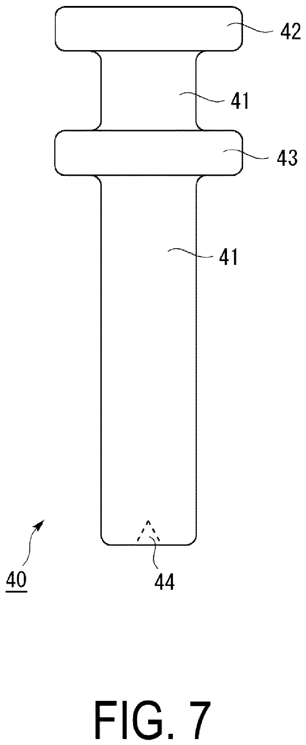

The internal space of the center portion 21a of the body 21 serves as a combustion chamber 20a into which a combustion product is discharged from the igniter 22. Furthermore, a male thread portion 26 is formed in a part of the outer surface of the center portion 21a. The male thread portion 26 is configured to mate with a female thread portion 32 of the attachment 30 described below. The effective lengths of the male thread portion 26 and the female thread portion 32 are determined to guarantee sufficient coupling force therebetween. The internal space of the distal end portion 21b adjacent to the center portion 21a is formed in a cylindrical shape in which a piston 40 is slidably provided and O rings 25 serving as a sealing member are also provided. The piston 40 is made of metal, has a shaft member 41, is provided with a first flange 42 on the base end side thereof, and is further provided with a second flange 43 in the vicinity of the first flange 42, as illustrated in FIG. 7. The first flange 42 and the second flange 43 have a disc shape, and have the same diameter. The O rings 25 include one disposed between the first flange 42 and the second flange 43 and one disposed on another side of the second flange 43. A recess portion 44 having a predetermined size is formed in a distal end surface of the shaft member 41. In a state where the piston 40 is disposed in the internal space of the distal end portion 21b before the actuation of the actuator 20, the first flange 42, which serves as a surface receiving pressure of the combustion product from the igniter 22, is exposed on side of the combustion chamber 20a, and the distal end of the shaft member 41 of the piston 40 is inserted into the opening 27.

Then, when the igniter 22 is activated and the combustion product is discharged into the combustion chamber 20a and thus the pressure therein rises, the first flange 42 receives the pressure, resulting in the piston 40 sliding toward the distal end side. Thus, the actuator 20 has a mechanism with the igniter 22 serving as an actuation source and the piston 40 serving as an output unit. Since the second flange 43 has a larger diameter than the opening 27, the distance by which the piston 40 can slide is limited. Thus, the distance by which the shaft member 41 of the piston 40 can protrude from the distal end surface of the distal end portion 21b of the body 21 is limited. Further, the piston 40 may be formed of a resin, and in such case, metal may be used together for a part to which heat resistance and pressure resistance are required.

Additionally, as an alternative mechanism to adjust the pressure applied to the piston 40, the combustion chamber 20a of the actuator 20 may be further provided with a gas generating agent that is burned by the combustion product from the igniter 22 to produce gas. The agent may be disposed, for example, at a location that may be exposed to the combustion product from the igniter 22. Further, as another method, the gas generating agent may be disposed in the igniter 22 as disclosed in WO 01/031282, JP 2003-25950 A, and the like. As one example of the gas generating agent, there may be exemplified a single base smokeless explosive formed of 98 mass% of nitrocellulose, 0.8 mass% of diphenylamine, and 1.2 mass% of potassium sulfate. Further, various types of gas generating agents used in a gas generator for an air bag and a gas generator for a seat belt pretensioner may be used. A combustion completion time period of the gas generating agent can be changed by adjusting a dimension, a size, a shape, and particularly, a surface shape of the gas generating agent at the time of being disposed in the combustion chamber 20a or the like. With this, the pressure applied to the piston 40 can be adjusted to a desired pressure.

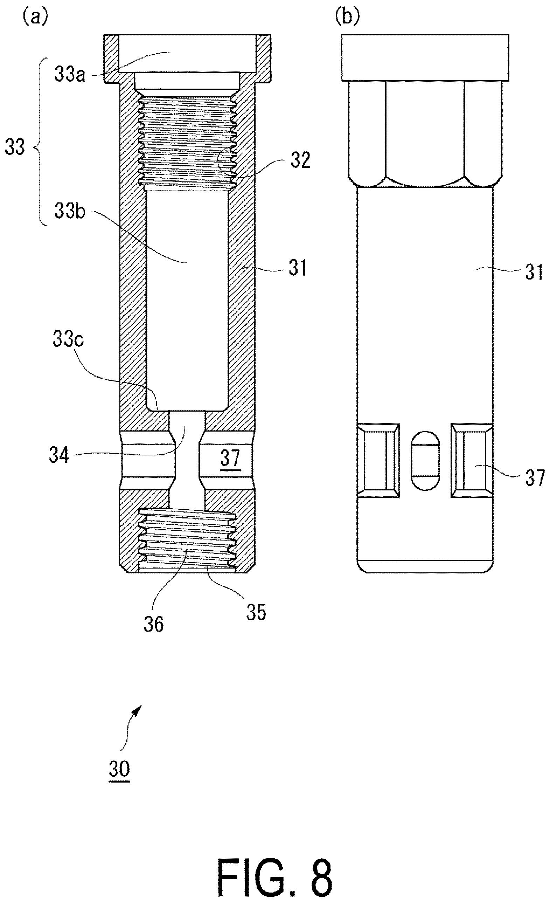

Next, the attachment 30 will be described based on FIG. 8. Note that FIG. 8 includes the diagram (a) on the left side that is a cross-sectional view of the attachment 30, and the diagram (b) on the right side that is an external view of the attachment 30. The attachment 30 is a member for attaching the actuator 20, the plunger 80, and the container 70 as illustrated in FIG. 5. For a body 31 of the attachment 30, nylon 6-12, polyarylate, polybutylene terephthalate, polyphenylene sulphide, a liquid crystal polymer, or the like, which are publicly known, may be used for example. Further, a filler such as glass fibers and glass filler may be contained in those resins. From 20 to 80 mass% of glass fibers may be contained in polybutylene terephthalate, from 20 to 80 mass% of glass fibers may be contained in polyphenylene sulphide, or from 20 to 80 mass% of minerals may be contained in a liquid crystal polymer.

The internal space of the body 31 includes a first region 33, extending from the base end side to the center, where the actuator 20 is disposed as illustrated in FIG. 5. The first region 33 includes a region 33a on the base end side where the base end portion 21c of the actuator 20 is generally positioned, and a region 33b on the distal end side of the first region 33 where the center portion 21a and the distal end portion 21b of the actuator 20 are generally positioned. The region 33b has a smaller diameter than the region 33a. The female thread portion 32 is disposed on the inner wall surface at a portion of the region 33b close to the region 33a. The female thread portion 32 is formed to engage with the male thread portion 26 provided on the center portion 21a of the actuator 20.

The internal space of the body 31 further includes a second region 34 in communication with the first region 33. The second region 34 is a region in which the plunger 80 is generally disposed as illustrated in FIG. 5, and is a hollow region formed in a cylindrical shape extending along the axial direction of the body 31. The second region 34 has one end in communication with the region 33b of the first region 33. The second region 34 has a diameter smaller that is smaller than the diameter of the region 33b, and enables a sliding movement of the plunger 80. A through hole 37 extends from a side outer surface of the attachment 30 to the second region 34, to be formed through the body 31. Through the through hole 37, the user can check the status (such as whether the injector assembly 10 is before or after being actuated, for example) of the plunger 80 in the injector assembly 10 from the outside (see FIG. 1).

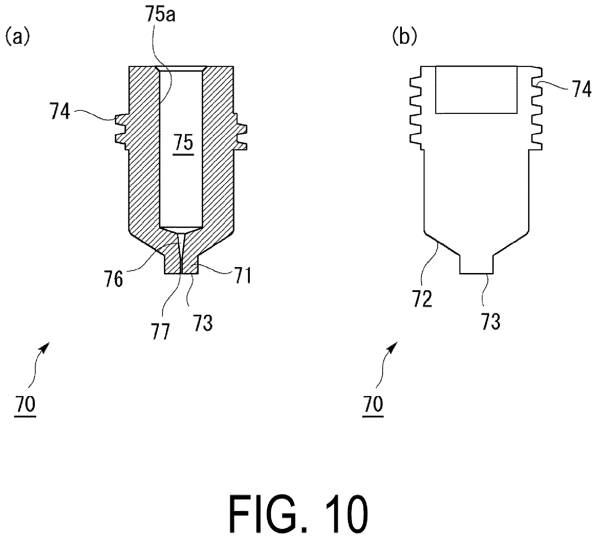

The internal space of the body 31 further includes a third region 35 in communication with the second region 34. The third region 35 is a region in which a part of the container 70 is generally disposed as illustrated in FIG. 5, and has one end in communication with the second region 34, and has the other end open to the distal end surface of the attachment 30. A female thread portion 36 for attachment to the container 70 is formed in the third region 35. The female thread portion 36 is screwed with a male thread portion 74 of the container 70 illustrated in FIG. 10 described below, whereby the attachment 30 and the container 70 are coupled to each other.

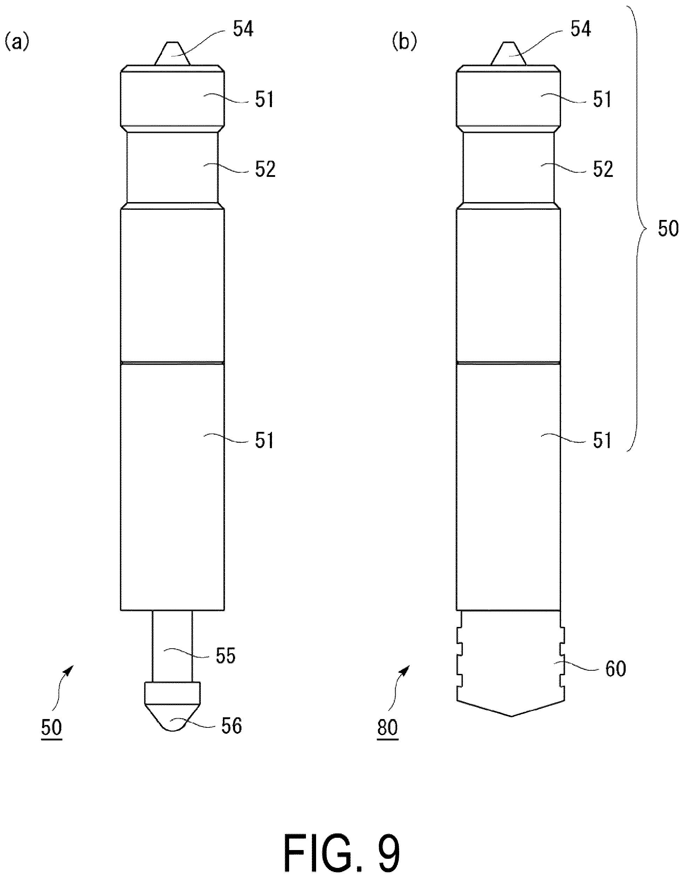

Next, the plunger 80 will be described based on FIG. 9. FIG. 9 includes the diagram (a) on the left side that is an external view of a plunger rod 50, which is one of the components of the plunger 80, and a diagram (b) on the right side that is an external view of the plunger 80. The plunger 80 is a member that pressurizes the ejection solution by energy received from the piston 40, and a resin material suitable for the pressurization (for example, a resin material similar to that used for the attachment 30) can be used for the plunger rod 50. The plunger rod 50 includes a shaft member 51, and has a base end side end surface provided with a protrusion 54. The protrusion 54 is shaped and sized to be capable of fitting in the recess portion 44 of the shaft member of the piston 40 of the actuator 20, when the plunger 80 is incorporated in the injector assembly 10. A reduced diameter portion 52 that has a diameter smaller than other portions of the shaft member 51 is provided in an intermediate portion of the shaft member 51 close to the base end.

Further, in the plunger rod 50, a protrusion 56 is provided to a distal end side of the shaft member 51 with a neck portion 55 with a smaller diameter than the shaft member 51 provided in between. The protrusion 56 is shaped like a weight to have a diameter being greater than the diameter of the neck portion 55 near a portion to be connected with the neck portion 55 and reducing toward the distal end side. The maximum diameter of the protrusion 56 is smaller than the diameter of the shaft member 51. A stopper portion 60 formed of an elastic member such as rubber is attached to the neck portion 55 and the protrusion 56, whereby the plunger 80 is formed (see FIG. 9(b)). An attachment hole (not illustrated) is formed in the stopper portion 60, and engages with the neck portion 55 and the protrusion 56, and the stopper portion 60 is less likely to be detached from the plunger rod 50.

Specific examples of materials of the stopper portion 60 include butyl rubber and silicon rubber. Further, there may be exemplified a styrene-based elastomer or a hydrogenated styrene-based elastomer, or a substance obtained by mixing a styrene-based elastomer or a hydrogenated styrene-based elastomer with polyolefin such as polyethylene, polypropylene, polybutene, and an α-olefin copolymer, oil such as liquid paraffin and process oil, or a powder inorganic substance such as talc, cast, and mica. Further, as the material of the stopper portion 60, a polyvinyl chloride-based elastomer, an olefin-based elastomer, a polyester-based elastomer, a polyamide-based elastomer, a polyurethane-based elastomer, various rubber materials (particularly, a vulcanized material) such as natural rubber, isoprene rubber, chloroprene rubber, nitrile butadiene rubber, and styrene butadiene rubber, or a mixture thereof may be employed. Furthermore, the stopper portion 60 pressurizes the ejection solution by sliding within the container 70 described below. Thus, a surface of the stopper portion 60 and an inner wall surface 75a of the accommodating space 75 of the container 70 may be coated or processed using various matters, to guarantee/adjust slidability between the stopper portion 60 and the inner wall surface 75a of the accommodating space 75 of the container 70. Examples of the coating agent may include polytetrafluoroethylene (PTFE), silicon oil, diamond-like carbon, nano diamond, and the like.

Next, the container 70 will be described based on FIG. 10. Note that FIG. 10 includes the diagram (a) on the left side that is a cross-sectional view of the container 70, and the diagram (b) on the right side that is an external view of the container 70. The container 70 is a member containing an ejection solution to be pressurized by the plunger 80, is a member that defines a flow path for ejecting the pressurized ejection solution to the target region through the ejection port, and corresponds to the housing part. In view of this, a resin material (a resin material of the same type as the attachment 30 for example) may be used for forming the container 70.

The container 70 includes an accommodating space 75, in which the stopper portion 60 of the plunger 80 are movable, accommodating the ejection solution, and a nozzle portion 71 including a flow path 76 connecting the accommodating space 75 to the ejection port 77 facing the outside of the container 70. The nozzle portion 71 has a columnar outer circumference on the distal end side. Note that in the injector assembly 10, as illustrated in FIG. 5, a positional relationship between the plunger 80 and the container 70 is determined such that the stopper portion 60 of the plunger 80 can slide within the accommodating space 75 in a direction toward the nozzle portion 71 (direction toward the distal end side). The ejection solution is sealed in a space defined by stopper portion 60 of the plunger 80 and the container 70. The flow path 76 of the container 70 opens in a distal end surface 73 of the nozzle portion 71, and the ejection port 77 is formed. Thus, when the plunger 80 slides within the accommodating space 75, the ejection solution accommodated in the accommodating space 75 is pressurized to be ejected from the ejection port 77 through the flow path 76.

The flow path 76 provided in the container 70 has a diameter smaller than the inner diameter of the accommodating space 75. With this configuration, the ejection solution that has been applied with a high pressure is ejected to the outside through the ejection port 77. The male thread portion 74 for attaching the container 70 to the attachment 30 is formed on the base end side of the container 70. The male thread portion 74 is screwed with the female thread portion 36 of the attachment 30.

Note that the profile on the distal end side of the stopper portion 60 of the plunger 80 is shaped to substantially match the profile of the inner wall surface 75a near a portion where the accommodating space 75 and the flow path 76 are connected to each other (the deepest part of the accommodating space 75). With this configuration, a smallest possible gap can be formed between the stopper portion 60 and the inner wall surface 75a of the container 70 when the plunger 80 slides for ejecting the ejection solution and reaches the deepest part of the accommodating space 75, whereby the ejection solution can be prevented from wastefully remaining in the accommodating space 75. However, the shape of the stopper portion 60 is not limited to a particular shape as long as desired effects can be obtained with the injector 1 according to the present embodiment.

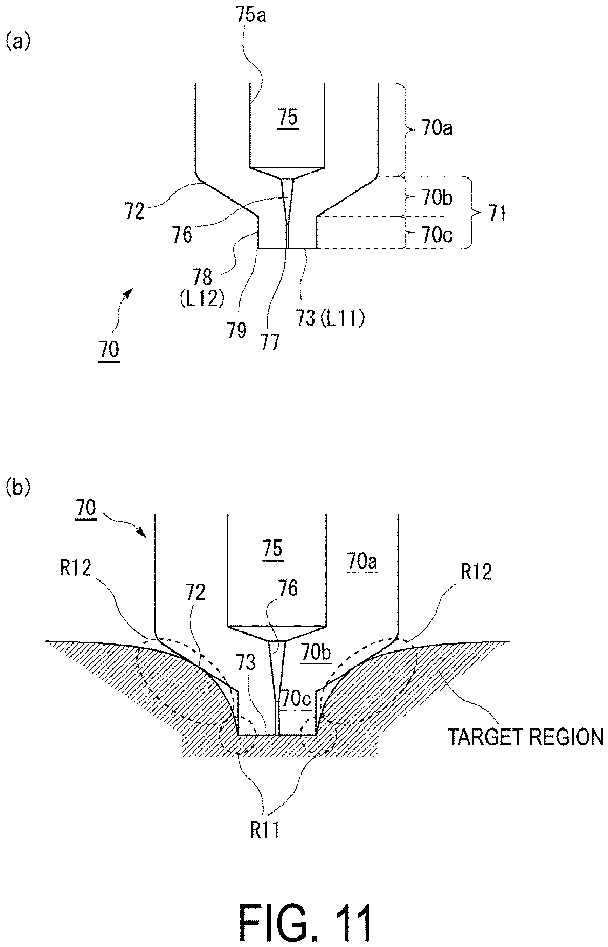

Now, the outer shape of the container 70 will be described based on FIG. 11. FIG. 11(a) on the upper side is an enlarged cross-sectional view of a distal end side portion of the container 70, taken along the axial direction of the container 70 as in FIG. 10(a). Note that in FIG. 11(a), the hatching representing the cross section is omitted. FIG. 11(b) on the lower side illustrates a state where the injector 1 is formed with the container 70 attached to the housing 2 (state illustrated in FIGS. 2 and 3) and in which the distal end surface 73 is in contact with the target region while being pressed thereagainst for ejecting the ejection solution from the injector 1.

Such a contact state between the distal end surface 73 and the target region largely affects the behavior of the ejection solution imparted with the ejection energy, within the target region, that is, how deep the ejection solution can reach within the target region, how the ejection solution spreads within the target region, and the like. In other words, the ejection solution cannot be favorably ejected from the needleless injector, unless the favorable contact state between the distal end surface 73 and the target region is achieved. In view of this, the container 70 of the present embodiment has the outer shape designed for achieving the favorable contact state between the distal end surface 73 and the target region. The shape will be described in detail.

As illustrated in FIG. 11(a), the container 70 includes: a housing part main body 70a incorporating an accommodating space 75 into which the stopper portion 60 of the plunger 80 can enter; a protrusion 70c located at a furthermost position of the container 70 on the distal end side and having a distal end surface formed to include the ejection port 77; and a connection part 70b connecting the housing part main body 70a and the protrusion 70c to each other. The connection part 70b and the protrusion 70c form the nozzle portion 71 described above, and the flow path 76 described above is formed through the connection part 70b and the protrusion 70c to communicate the accommodating space 75 and the ejection port 77 to each other.

The housing part main body 70a and the protrusion 70c are substantially columnar, and have the center axes matching each other and matching that of the container 70. Furthermore, the protrusion 70c is formed to have an outer diameter smaller than that of the housing part main body 70a. Thus, the connection part 70b has an outer surface 72 of an umbrella shape with a diameter increasing toward the housing part main body 70a from the protrusion 70c. In other words, the outer surface of the connection part 70b is an annular inclined surface 72 that is inclined with respect to the center axis of the container 70 in the cross section illustrated in FIG. 11(a). In the present embodiment, the inclined surface 72 has no portion protruding toward the distal end side of the container 70, and continuously and linearly connects the end portions of the protrusion 70c and the housing part main body 70a to each other.

Furthermore, in the state in which the injector 1 is assembled as illustrated in FIG. 2, the distal end surface 73 and a side surface 78 of the protrusion 70c as well as the inclined surface 72 of the connection part 70b are exposed to the outside of the housing 2. With the protrusion 70c and the connection part 70b formed as described above in the container 70, the protrusion 70c at a furthermost position of the container 70 on the distal end side is formed to be the thinnest and is connected to the housing part main body 70a with the inclined surface 72 illustrated in FIG. 11(a) provided in between. Thus, a space without a component of the injector 1 can be secured on the distal end side of the inclined surface 72. As a result, the user attempting to bring the distal end surface 73 of the container 70 into contact with the target region can easily visually recognize the position of the protrusion 70c, whereby extremely high operability of the injector can be achieved.

As illustrated in FIG. 11(b), when the injector 1 is pressed against the target region with the distal end surface 73 being in contact with the target region, the target region tends to deform with the pressed portion recessed and the target region therearound risen. In such a case, with the above-described space secured on the distal end side of the container 70, the raised target region can be guided into the space (the space defined by a dotted line R12 illustrated in FIG. 11(b)). As a result, the container 70 forming the injector 1 and the target region can be prevented from being in excessive contact with each other, whereby the favorable contact state between the distal end surface 73 and the target region can be prevented from being compromised by the interference between the container 70 and the target region. In particular, the interference between the container 70 and the target region can be suppressed, even when the distal end surface 73 of the container 70 comes into contact with the target region while being inclined.

Furthermore, in the protrusion 70c of the container 70, an outer circumference edge 79 of the distal end surface 73 where the distal end surface 73 and the side surface 78 intersect is formed as a corner part including no chamfered part. More specifically, the outer circumference edge 79 is represented by an intersection point formed as a geometrical intersection between a first straight line L11 defining the distal end surface 73 and a second straight line L12 defining the side surface 78 in the cross section illustrated in FIG. 11(a). In the present embodiment, the outer circumference edge 79 includes no geometrical element other than the intersection point between the first straight line L11 and the second straight line L12. Thus, the outer circumference edge 79 does not include what is known as an R chamfered part or a C chamfered part, and thus is formed as a sharp corner part. As an example, an intersection angle between the first straight line L11 and the second straight line L12 is approximately 90 degrees.

With the outer circumference edge 79 of the distal end surface 73 formed in this manner, when the distal end surface 73 is brought into contact with the target region as illustrated in FIG. 11(b), the outer circumference edge 79 favorably bites into the target region (see the region defined by a dotted line R11 illustrated in FIG. 11(b)). As a result, a more airtight contact state between the distal end surface 73 and the target region is achieved, and the injector 1 is stably fixed to the target region via the container 70. Thus, the administration performance of the ejection solution from the injector 1 can be improved. In other words, with the injector 1 favorably supported with the outer circumference edge 79, the ejection solution can be ejected with a smaller amount of pressing force required for fixing the injector 1 with respect to the target region. This leads to reduction of the interference between the container 70 and the target region as a result of rising of the target region due to the pressing force, resulting in improvement of the operability and the ejection solution administration performance of the injector 1.

In the present embodiment, a part of the target region risen by the pressing force may be in contact with the inclined surface 72 of the container 70, in a state where the distal end surface 73 is in contact with the target region. Even in such a case, the container 70 can be prevented from receiving excessively large counterforce from the target region because the space can be secured on the distal end side of the inclined surface as described above. Thus, when the target region is brought into contact with the inclined surface 72, the target region thus brought into contact can favorably support the injector 1 via the container 70 with moderate counterforce, whereby the stability of the injector 1 can be improved, and the administration performance of the injector 1 can be improved.

Based on the configuration of the parts of the injector 1 described above, the assembly of the injector assembly 10 will be described below. In a state where the stopper portion 60 of the plunger 80 is inserted to the deepest part of the accommodating space 75 of the container 70, the plunger 80 is retracted with the ejection port 77 of the container 70 in communication with the ejection solution. The stopper portion 60 and the inner wall surface 75a of the accommodating space 75 are suitably in close contact with each other, the retraction action will produce negative pressure in the accommodating space. Thus, the accommodating space 75 can be filled with the ejection solution through the ejection port 77. In this process, the plunger 80 is retracted to an extent enough for making the part of the plunger 80 (plunger rod 50) protruding from the container 70 pass through the second region 34 to reach the first region 33 (the region 33b illustrated in FIG. 8), when the container 70 is attached to the attachment 30 in this state.

After the container 70 filled with ejection solution in the accommodating space 75 is attached to the attachment 30, the actuator 20 is inserted to the attachment 30 from the side of the first region 33. The actuator 20 is inserted until the distal end surface of its distal end portion 21b comes into contact with a distal end surface 33c of the region 33b of the attachment 30 (see FIG. 8). Then, in this process, the male thread portion 26 provided to the center portion 21a of the actuator 20 is screwed with the female thread portion 32 of the attachment 30, whereby the actuator 20 and the attachment 30 are suitably coupled to each other. Furthermore, in this process, the recess portion 44 of the shaft member 41 of the piston 40, which is incorporated in the actuator 20, engages with the protrusion 54 of the shaft member 51 of the plunger 80, and the plunger 80 is pushed by the piston 40 toward the distal end side. Note that, a fixing force of the piston 40 in the distal end portion 21b of the actuator 20 is set to an extent that the piston 40 can slide in the distal end portion 21b in a sufficiently smooth manner by a pressure received from the combustion product produced by the igniter 22, and to an extent that the piston 40 can suitably resist force received from the plunger 80 such that the position of the piston 40 is not displaced when the injector assembly 10 is assembled. Alternatively, a stopper may be formed at an intended position of the piston 40, and the top surface of the first flange 42 of the piston 40 faces the combustion chamber 20a of the actuator 20 and is not displaced toward the combustion chamber 20a as illustrated in FIG. 6.

Thus, when the actuator 20 is attached to the attachment 30 to which the container 70 and plunger 80 are attached as described above, the plunger 80 is pushed to move from the piston 40 toward the distal end side, whereby the plunger 80 is positioned at a predetermined position within the container 70. Note that, in response to pressing of the plunger 80, a part of the ejection solution is discharged from the ejection port 77.

When the plunger 80 is thus positioned at the final position as described above, formation of the injector assembly 10 is completed. In this injector assembly 10, the position of the stopper portion 60 of the plunger 80 in the accommodating space 75 of the container 70 is mechanically determined. The final position of the stopper portion 60 is a position uniquely determined in the injector assembly 10, and hence an amount of the ejection solution that is finally stored in the accommodating space 75 in the injector assembly 10 can be a predetermined amount determined in advance.

The injector assembly 10 thus configured can be loaded into the housing 2 with the ignition pin 22b of the igniter 22 fitted into the socket 7 on the housing 2, whereby the injector 1 is prepared to be usable (see FIGS. 1 to 3). Then, when predetermined operations are performed on the injector 1 using the first switch 5 and the second switch 6 in the usable state, whereby the ejection solution is ejected to the target region.

### Reference Signs List

1 Injector
2 Housing
2a Grip portion
5 First switch
6 Second switch
10 Injector assembly
20 Actuator
21 Body
22 Igniter
30 Attachment
31 Body
40 Piston
50 Plunger rod
51 Shaft member
52 Reduced diameter portion
60 Stopper portion
70 Container
70a Housing part main body
70b connection part
70c Protrusion
71 Nozzle portion
72 Inclined surface
73 Distal end surface
75 Accommodating space
76 Flow path
77 Ejection port
78 Side surface
79 Outer circumference edge
80 Plunger

## Claims

1. A needleless injector (1) that ejects an intended injection substance to a target region without using an injection needle, the needleless injector (1) comprising:
a housing part including an accommodating space (75) in which the intended injection substance is accommodated, the housing part defining a flow path (76) to allow the intended injection substance to be ejected to the target region through an ejection port (77);
a driving part that imparts ejection energy for ejecting the intended injection substance;
a pressurizing unit that pressurizes the intended injection substance accommodated in the accommodating space (75), upon being imparted with the ejection energy; and
an injector enclosure (2) to which the housing part, the driving part, and the pressurizing unit are attached to form the needleless injector (1), wherein
the housing part includes:
a housing part main body (70a) incorporating the accommodating space (75);
a protrusion (70c) that protrudes in an axial direction of the housing part on a distal end side of the housing part, the protrusion (70c) including a distal end surface (73) in which the ejection port (77) is formed, the protrusion (70c) having an outer diameter that is smaller than an outer diameter of the housing part main body (70a), the distal end surface (73) having at least a part defined by a first straight line and the protrusion (70c) having a side surface (78) defined by a second straight line in a cross section taken along the axial direction of the housing part, the distal end surface (73) having an outer circumference edge (79) represented by an intersection point between the first straight line and the second straight line; and
a connection part (70b) connecting the protrusion (70c) and the housing part main body (70a) to each other, and having an outer surface forming an annular inclined surface (72) that is inclined with respect to a center axis of the housing part and continues from an end portion of the protrusion (70c) to an end portion of the housing part main body (70a),
the flow path (76) is formed in the protrusion (70c) and the connection part (70b) to communicate the ejection port (77) and the accommodating space (75) to each other, and
in a state where the housing part is attached to the injector enclosure (2), the distal end surface (73) and the side surface (78) of the protrusion (70c) and the inclined surface (72) of the connection part (70b) are exposed to outside of the injector enclosure (2)
**characterized in that**
the inclined surface (72) has no portion protruding toward the distal end side of the housing part, and continuously and linearly connects the end portions of the protrusion (70c) and the housing part main body (70a) to each other, in the cross section taken along the axial direction of the housing part.

2. The needleless injector (1) according to claim 1, wherein an angle formed between the first straight line and the second straight line is approximately 90 degrees in the cross section taken along the axial direction of the housing part.

3. The needleless injector (1) according to claim 1 or 2, wherein the inclined surface (72) is defined by a third straight line in the cross section taken along the axial direction of the housing part.

## Patentansprüche

1. Ein nadelloser Injektor (1), der eine beabsichtigte Injektionssubstanz in einen Zielbereich ausstößt, ohne eine Injektionsnadel zu verwenden, wobei der nadellose Injektor (1) umfasst:
einen Gehäuseteil, der einen Aufnahmeraum (75) enthält, in dem die beabsichtigte Injektionssubstanz aufgenommen ist, wobei der Gehäuseteil einen Strömungsweg (76) definiert, um zu ermöglichen, dass die beabsichtigte Injektionssubstanz durch eine Ausstoßöffnung (77) zu dem Zielbereich ausgestoßen wird;
einen Antriebsteil, der Ausstoßenergie zum Ausstoßen der beabsichtigten Injektionssubstanz aufbringt;
eine Druckbeaufschlagungseinheit, die die beabsichtigte Injektionssubstanz, die in dem Aufnahmeraum (75) aufgenommen ist, mit Druck beaufschlagt, in Erwiderung auf das Aufbringen der Ausstoßenergie darauf; und
eine Injektoreinfassung (2), an der der Gehäuseteil, der Antriebsteil und die Druckbeaufschlagungseinheit angebracht sind, um den nadellosen Injektor (1) zu bilden, wobei
der Gehäuseteil enthält:
einen Gehäuseteilhauptkörper (70a), der den Aufnahmeraum (75) enthält;
einen Vorsprung (70c), der in einer axialen Richtung des Gehäuseteils auf einer distalen Endseite des Gehäuseteils vorsteht, wobei der Vorsprung (70c) eine distale Endfläche (73) enthält, in der die Ausstoßöffnung (77) ausgebildet ist, wobei der Vorsprung (70c) einen Außendurchmesser hat, der kleiner als ein Außendurchmesser des Gehäuseteilhauptkörpers (70a) ist, die distale Endfläche (73) zumindest einen Teil aufweist, der durch eine erste gerade Linie definiert ist, und der Vorsprung (70c) eine Seitenfläche (78) aufweist, die durch eine zweite gerade Linie in einem entlang der axialen Richtung des Gehäuseteils genommenen Querschnitt definiert ist, die distale Endfläche (73) eine Außenumfangskante (79) aufweist, die durch einen Schnittpunkt zwischen der ersten geraden Linie und der zweiten geraden Linie repräsentiert wird; und
ein Verbindungsteil (70b), der den Vorsprung (70c) und den Gehäuseteilhauptkörper (70a) miteinander verbindet und eine Außenfläche aufweist, die eine ringförmige geneigte Fläche (72) bildet, die in Bezug auf eine Mittelachse des Gehäuseteils geneigt ist und sich von einem Endabschnitt des Vorsprungs (70c) zu einem Endabschnitt des Gehäuseteilhauptkörpers (70a) fortsetzt,
der Strömungsweg (76) in dem Vorsprung (70c) und dem Verbindungsteil (70b) ausgebildet ist, um die Ausstoßöffnung (77) und den Aufnahmeraum (75) miteinander zu verbinden, und in einem Zustand, in dem der Gehäuseteil an der Injektoreinfassung (2) angebracht ist, die distale Endfläche (73) und die Seitenfläche (78) des Vorsprungs (70c) und die geneigte Fläche (72) des Verbindungsteils (70b) zur Außenseite der Injektoreinfassung (2) freigelegt sind, **dadurch gekennzeichnet, dass**
die geneigte Fläche (72) keinen Abschnitt aufweist, der in Richtung der distalen Endseite des Gehäuseteils vorsteht, und die Endabschnitte des Vorsprungs (70c) und des Gehäuseteilhauptkörpers (70a) im entlang der axialen Richtung des Gehäuseteils genommenen Querschnitt kontinuierlich und linear miteinander verbindet.

2. Der nadellose Injektor (1) nach Anspruch 1, wobei ein zwischen der ersten geraden Linie und der zweiten geraden Linie gebildeter Winkel im entlang der axialen Richtung des Gehäuseteils genommenen Querschnitt etwa 90 Grad beträgt.

3. Der nadellose Injektor (1) nach Anspruch 1 oder 2, wobei die geneigte Fläche (72) durch eine dritte gerade Linie im entlang der axialen Richtung des Gehäuseteils genommenen Querschnitt definiert ist.

## Revendications

1. Injecteur sans aiguille (1) qui éjecte une substance d'injection prévue à une région cible sans utilisation d'une aiguille d'injection, l'injecteur sans aiguille (1) comprenant :
une partie logement incluant un espace d'accueil (75) dans lequel la substance d'injection prévue est logée, la partie logement définissant un trajet d'écoulement (76) pour permettre à la substance d'injection prévue d'être éjectée au niveau de la région cible à travers un orifice d'éjection (77) ;
une partie d'entraînement qui confère de l'énergie d'éjection pour éjecter la substance d'injection prévue ;
une unité de pressurisation qui met la substance d'injection prévue sous pression logée dans l'espace d'accueil (75), lorsqu'il lui a été conférée l'énergie d'éjection ; et
une enceinte d'injecteur (2) à laquelle la partie logement, la partie d'entraînement, et l'unité de pressurisation sont fixées pour former l'injecteur sans aiguille (1), dans lequel
la partie logement inclut :
un corps principal de partie logement (70a) incorporant l'espace d'accueil (75) ;
une protubérance (70c) qui fait saillie dans un sens axial de la partie logement sur un côté d'extrémité distale de la partie logement, la protubérance (70c) incluant une surface d'extrémité distale (73) dans laquelle l'orifice d'éjection (77) est formé, la protubérance (70c) ayant un diamètre externe qui est inférieur à un diamètre externe du corps principal de partie logement (70a), la surface d'extrémité distale (73) ayant au moins une partie définie par une première ligne droite et la protubérance (70c) ayant une surface latérale (78) définie par une seconde ligne droite dans une section transversale prise le long du sens axial de la partie logement, la surface d'extrémité distale (73) ayant un bord de circonférence (79) représenté par un point d'intersection entre la première ligne droite et la seconde ligne droite ; et
une partie de raccordement (70b) raccordant la protubérance (70c) et le corps principal de partie logement (70a) l'un à l'autre, et ayant une surface externe formant une surface inclinée annulaire (72) qui est inclinée par rapport à un axe central de la partie logement et qui continue depuis une portion d'extrémité de la protubérance (70c) jusqu'à une portion d'extrémité du corps principal de partie logement (70a),
le trajet d'écoulement (76) est formé dans la protubérance (70c) et la partie de raccordement (70b) pour faire communiquer l'orifice d'éjection (77) et l'espace d'accueil (75) l'un avec l'autre, et
dans un état où la partie logement est fixée à l'enceinte d'injecteur (2), la surface d'extrémité distale (73) et la surface latérale (78) de la protubérance (70c) et la surface inclinée (72) de la partie de raccordement (70b) sont exposées vers l'extérieur de l'enceinte d'injecteur (2)
**caractérisé en ce que** la surface inclinée (72) ne présente aucune portion faisant saillie vers le côté d'extrémité distale de la partie logement, et raccorde de manière continue et linéaire les portions d'extrémité de la protubérance (70c) et le corps principal de la partie logement (70a) l'un à l'autre, dans la section transversale prise le long du sens axial de la partie logement.

2. Injecteur sans aiguille (1) selon la revendication 1, dans lequel un angle formé entre la première ligne droite et la seconde ligne droite est d'approximativement 90 degrés dans la section transversale prise le long du sens axial de la partie logement.

3. Injecteur sans aiguille (1) selon la revendication 1 ou 2, dans lequel la surface inclinée (72) est définie par une troisième ligne droite dans la section transversale prise le long du sens axial de la partie logement.
